(19)

**Europäisches Patentamt**
**European Patent Office**
**Office européen des brevets**

(11)  **EP 4 751 646 A1**

(12) **EUROPEAN PATENT APPLICATION**

(43) Date of publication:
03.06.2026  Bulletin 2026/23

(21) Application number: 24216698.1

(22) Date of filing: **29.11.2024**

(51) International Patent Classification (IPC):
*A61B 5/0537* (2021.01)    *G16H 50/20* (2018.01)
*A61B 5/0538* (2021.01)    *A61B 17/28* (2006.01)
*A61B 5/0535* (2021.01)    *A61B 17/29* (2006.01)

(52) Cooperative Patent Classification (CPC):
A61B 5/7257; A61B 5/0084; A61B 5/0537;
A61B 5/0538; A61B 5/4836; A61B 5/4872;
A61B 5/6847; A61B 5/6876; A61B 5/7221;
A61B 5/7267; A61B 17/28; G16H 10/40;
G16H 30/20; G16H 30/40; G16H 40/63;    (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Stichting IMEC Nederland**
**5656 AE Eindhoven (NL)**

(72) Inventors:
• **PETKOS, Konstantinos**
 **5615 AE Eindhoven (NL)**
• **HOOGEVEEN, Maartje**
 **5631 CA Eindhoven (NL)**

• **LANGEREIS, Geert**
 **5611 HX Eindhoven (NL)**
• **BOERS, Heleen**
 **5651 HR Eindhoven (NL)**
• **BEUTEL, Fabian**
 **40547 Duesseldorf, (DE)**
• **LINDEBOOM, Lucas**
 **9991 AA Middelstum (NL)**
• **SPLIETHOFF, Jarich**
 **3971 AA Driebergen-Rijsenburg (NL)**

(74) Representative: **AWA Sweden AB**
 **Matrosgatan 1**
 **Box 5117**
 **200 71 Malmö (SE)**

(54)    **A SYSTEM FOR CHARACTERIZATION OF TISSUE OF A SUBJECT**

(57)    According to an aspect of the present inventive concept there is provided a system for characterization of tissue of a subject, comprising a bioimpedance sensing arrangement, configured to sense an impedance of the tissue for forming impedance data, an optical property sensing arrangement, configured to sense an optical property of the tissue, a decision unit, configured to receive input data, process the input data to perform a characterization of the tissue, and estimate a confidence value for the characterization of the tissue, wherein the input data comprises the impedance data, and a control unit, configured to determine whether the confidence value subceeds a threshold and, based on the determination that the confidence value subceeds the threshold, activate the optical property sensing arrangement for acquiring further information for characterization of the tissue. According to another aspect, there is provided a method for controlling a system for characterization of tissue of a subject

Fig. 1

**(Cont. next page)**

(52) Cooperative Patent Classification (CPC): (Cont.)
**G16H 50/20; G16H 50/70;** A61B 5/0095;
A61B 5/0535; A61B 5/489; A61B 2017/00026;
A61B 2017/00057; A61B 2017/2948;
A61B 2217/002; A61B 2505/05; A61B 2562/0209;
A61B 2562/0238; A61B 2562/063

## Description

Technical field

The present description relates to characterization of tissue of a subject. In particular, the present description relates to a system for characterization of tissue of a subject and a method for controlling a system for characterization of tissue of a subject.

Background

Tissue characterization is a crucial process in both medical and biological research. It involves analyzing the physical, chemical, and biological properties of tissues to understand their structure, function, and pathology. This process can include a variety of techniques such as histology, immunohistochemistry, and molecular analysis.

One of the primary reasons tissue characterization is important is its role in diagnosing diseases. By examining tissue samples, pathologists can identify abnormalities that indicate conditions like cancer, infections, or inflammatory diseases. This helps in making accurate diagnoses and determining the most effective treatment plans.

Moreover, tissue characterization is vital in research and development. It allows scientists to study the effects of new drugs or treatments on tissues, providing insights into their efficacy and safety. This is particularly important in the development of personalized medicine, where treatments are tailored to the individual characteristics of a patient's tissues.

Additionally, understanding tissue properties is essential in regenerative medicine and tissue engineering. By characterizing tissues, researchers can develop better biomaterials and scaffolds that mimic the natural environment of cells, promoting tissue repair and regeneration.

Presently, clinicians either use portable medical devices, which cannot provide a fully accurate bioelectrical profile of the tissues under examination or advanced surgical imaging methodologies for providing more accurate characterization.

There is an ongoing need for methods and devices that can accurately guide surgeons during surgery and detect and characterize medical pathologies in the body of a subject. There is currently a particular need for the development of versatile systems that can provide safe, reliable, and low-cost analysis of such pathologies.

Those medical pathologies include cancerous tumors and lesions, bone fractures, and diseased tissue in the cardiopulmonary or neurological systems. Moreover, the problem of accurately identifying a vessel during surgery, aiming at properly sealing it, has not been fully addressed yet.

This fact necessitates the development of precise and inexpensive techniques that can identify vessels, which are often hidden inside complex bundles comprising various types of other tissues.

Summary

An objective of the present description is to provide safe, reliable, and low-cost systems for characterization of tissue of a subject and methods for controlling said systems.

This and other objectives are at least partly met by the invention as defined in the independent claims. Preferred embodiments are set out in the dependent claims.

According to a first aspect, there is provided a system for characterization of tissue of a subject, the system comprising a bioimpedance sensing arrangement, configured to be arranged within the subject, and configured to sense an impedance of the tissue for forming impedance data, an optical property sensing arrangement, configured to be arranged within the subject, and configured to sense an optical property of the tissue for forming optical property data, a control unit, configured to initially initiate a first sensor measurement, wherein the control unit is configured to, in the first sensor measurement, activate a first sensing arrangement selected from the bioimpedance sensing arrangement and the optical property sensing arrangement, and a decision unit, configured to receive input data from the first sensing arrangement, process the input data to perform a characterization of the tissue, and estimate a confidence value for the characterization of the tissue, wherein the control unit, is further configured to determine whether the confidence value subceeds a threshold and, based on the determination that the confidence value subceeds the threshold, initiate a second sensor measurement, wherein the control unit is configured to, in the second sensor measurement, activate a second sensing arrangement selected from the bioimpedance sensing arrangement and the optical property sensing arrangement for acquiring further information for characterization of the tissue, wherein the second sensing arrangement is non-selected in the first sensor measurement.

The system may be used for characterization of tissue of a subject, wherein the subject may be a living being. It should be realized that the system may be used for characterization of tissue of a human being but that the system may likewise be used for characterization of tissue of an animal.

As used herein, the term "characterization" may be understood to be mean discrimination, evaluation, analysis, or identification. In other words, "characterization" may be understood as analysis of data such that the tissue type may be identified, and further discrimination between tissue types may be achieved.

Tissues are groups of cells that work together to perform specific functions in the body. There are four main types of tissues in humans and animals. Epithelial tissue, which covers body surfaces and lines cavities, connective tissue, which supports and binds other tissues (including bone, blood, and fat), muscle tissue, which is responsible for movement, and nervous tissue, which transmits signals and processes information.

Each type of tissue has a unique structure and function, contributing to the overall health and functionality of the subject.

As used herein, the term "tissue" or "tissue of a subject" may be construed as a tissue or a plurality of tissues, e.g., an organ. Thus, it should be realized that "tissue" may include complex structures formed by one or more tissue types, such as vessels, organs, tumors, other pathological growths, etc.. Thus, characterization of tissue may not only relate to characterization of a tissue type but may also relate to characterization of a complex structure of tissue.

The bioimpedance sensing arrangement is configured to be arranged within the subject, and configured to sense an impedance of the tissue for forming impedance data.

As used herein, "impedance" may be understood to refer to bioimpedance. Bioimpedance refers to the measurement of the electrical impedance of biological tissues. It involves assessing how tissues resist and react to an electrical current, providing insights into the composition and properties of the tissue. Bioimpedance may be measured by sending an electrical current through the tissue and detecting how the tissue resists or reacts to it. However, it should be realized that bioimpedance may also be measured by applying a voltage across a tissue portion and detecting an induced current.

The bioimpedance sensing arrangement may comprise a plurality of electrodes. The bioimpedance sensing arrangement may comprise at least two electrodes, which may be arranged in a bipolar arrangement in contact with the tissue for providing an electrical signal and sensing a response. However, the bioimpedance sensing arrangement may preferably comprise at least four electrodes, which may be arranged in a tetrapolar arrangement in contact with the tissue to provide an electrical signal using a first pair of electrodes and to sense a response signal using a second pair of electrodes.

Each of the electrodes of the bioimpedance sensing arrangement may be configured to both provide a current and to sense a voltage response. In other words, each of the electrodes of the bioimpedance sensing arrangement may be configured to act as both stimulating electrode and recording electrode, and may further be configured to switch between stimulating and recording.

The bioimpedance sensing arrangement may be configured to perform at least one of a transmissive recording, i.e., a measurement of bioimpedance through a tissue segment based on electrodes being arranged on opposite sides of the tissue segment, or a reflective recording, i.e., a measurement of bioimpedance mainly along a surface of a tissue segment based on electrodes being arranged on a same side of the tissue segment. Preferably, the bioimpedance sensing arrangement is configured to perform both a transmissive recording and a reflective recording.

The optical property sensing arrangement is configured to be arranged within the subject, and configured to sense an optical property of the tissue.

The optical property of the tissue may for example be related to absorption or scattering of light by the tissue. The optical property of the tissue may be measured by sending light through the tissue and detecting how the tissue interacts with the light. For example, the intensity of the light after propagation through the tissue may be recorded.

Alternatively, soundwaves as a result of light absorption of the tissue may be detected. Thus, the optical property sensing arrangement may be configured to perform photo-acoustic detection. The light sent through the tissue may be modulated by a modulation frequency. As the tissue absorbs part of the light sent through the tissue, the tissue may cause a vibration at the modulation frequency, which may be detected as a soundwave.

It should also be realized that the optical property of the tissue may be related to an induced emission of light by the tissue, wherein the light emitted by the tissue may have a same or a different wavelength than the light received by the tissue. For example, the optical property of the tissue may relate to emission of fluorescence light by the tissue.

As used herein, light may relate to visible light but may also or alternatively relate to other parts of the electro-magnetic spectrum, such as infrared light or ultraviolet light.

The optical property sensing arrangement may comprise a plurality of optical components. Each optical component may be configured to send light into tissue and/or receive light from tissue. The optical property sensing arrangement may comprise a light source being configured to send light into tissue and a light sensor being configured to receive and detect light from the tissue. Thus, the light source and the light sensor may form optical components of the optical property sensing arrangement. However, it should be realized that the light source and the light sensor may not necessarily be arranged in close relation to tissue. Rather, the optical property sensing arrangement may comprise waveguides for guiding light from a light source to an optical component for outputting light into tissue and/or for guiding light received from tissue by an optical component to a light sensor. The optical components of the optical property sensing arrangement may thus be formed by interfaces for sending light into tissue and receiving light from tissue.

Each of the optical components of the optical property sensing arrangement may be configured to both send light into tissue and to receive an optical response. For instance, the same waveguide may be connected to both the light source and the light sensor such that light may be sent into tissue and received from tissue in the same position. However, the optical property sensing arrangement may alternatively comprise optical components dedicated to sending light into tissue or receiving light from the tissue, respectively.

In another example, the optical property sensing arrangement may comprise a light source and an acoustic sensor. The optical property sensing arrangement may comprise a light source being configured to send light into tissue and an acoustic sensor being configured to detect soundwaves from the tissue caused by the absorption of the light in the tissue.

The acoustic sensor may for example be an ultrasound transducer or an ultrasound receiver.

The plurality of components of the optical property sensing arrangement may comprise a combination of light sources, light sensors and acoustic sensors. Hence, it is a realization that the components may comprise a variety of components that may be used to detect or determine an optical property of the tissue. The components may be optical components, such as light sources and light sensors, but may also be an acoustic component, used to detect a soundwave formed by an optical property of the tissue.

It is a realization that the optical property may be a tissue property corresponding to an optical property of the tissue. For example, the tissue property may be a thermal property, corresponding to the absorption of light in the tissue.

The optical property sensing arrangement may be configured to perform at least one of a transmissive recording, i.e., a measurement of the optical property through a tissue segment based on optical components being arranged on opposite sides of the tissue segment, or a reflective recording, i.e., a measurement of the optical property mainly along a surface of a tissue segment based on optical components being arranged on a same side of the tissue segment. Preferably, the optical property sensing arrangement is configured to perform both a transmissive recording and a reflective recording.

The system further comprises a control unit configured to initially initiate a first sensor measurement. The first sensor measurement should be understood to be a first measurement of either the impedance of the tissue or the optical property of the tissue, using either the impedance sensing arrangement or the optical property sensing arrangement, respectively.

The control unit is further configured to, in the first sensor measurement, activate a first sensing arrangement selected from the bioimpedance sensing arrangement and the optical property sensing arrangement.

Thus, the first sensing arrangement may be the bioimpedance sensing arrangement or the optical property sensing arrangement, whichever is activated first.

The decision unit is configured to receive input data. The input data may comprise the impedance data. The input data may comprise the optical property data.

It is a realization that the impedance data or the optical property data may be pre-processed before forming part of the input data. This pre processing may for example be filtering.

The decision unit is further configured to process the input data to perform a characterization of the tissue. The processing performed by the decision unit may include extracting or identifying specific properties or identifiers of the input data. The properties may for example be the phase or magnitude of the impedance data. In certain examples a Fast Fourier Transform (FFT) is used to extract said properties.

The characterization may be performed by comparing the extracted properties with known tissue properties. In other examples, a classifier is used to characterize the tissue.

The decision unit is further configured to estimate a confidence value for the characterization of the tissue. The term "confidence value" as used herein refers to a statistical measure that quantifies the reliability of the characterization of tissue. The confidence value may be expressed as a percentage, indicating the likelihood that the characterization is accurate. A higher confidence value denotes greater reliability, while a lower confidence value indicates an increased uncertainty.

The control unit is further configured to determine whether the confidence value subceeds a threshold.

The threshold may be a predetermined threshold or determined on a case to case basis. In general, a confidence value of 95% or higher is often considered strong, indicating a high level of reliability. For more critical applications even higher confidence values may be required to ensure accuracy and minimize risk of mischaracterizations. Thus, it should be realized that the threshold may depend at least on the type of tissue characterized or the reason for characterization. In certain examples, a confidence value of 90% may be considered to have a high level of reliability, in others a confidence value of 98% may be considered to have a high level of reliability.

The threshold may therefore be determined based on the specific requirements and acceptable risk levels of the application in question.

The control unit is further configured to, based on the determination that the confidence value subceeds the threshold, initiate a second sensor measurement for acquiring further information for characterization of the tissue.

The second sensor measurement should be understood to be a second measurement of either the impedance of the tissue or the optical property of the tissue, using either the impedance sensing arrangement or the optical property sensing arrangement, respectively.

The control unit is further configured to, in the second sensor measurement, activate a second sensing arrangement selected from the bioimpedance sensing arrangement and the optical property sensing arrangement for acquiring further information for characterization of the tissue.

In other words, if the impedance sensing arrangement is activated in the first sensor measurement, the optical property sensing arrangement is activated in the second sensor measurement.

Similarly, if the optical property sensing arrangement is activated in the first sensor measurement, the bioimpedance sensing arrangement is activated in the second sensor measurement.

Hence, the second sensing arrangement is non-selected in the first sensor measurement.

After activation of the second sensing arrangement a second recording may be performed.

The input data may thus, after the second recording, further comprise the bioimpedance data or the optical property data such that the input data comprises both bioimpedance data and optical property data, and the decision unit may further be configured to process the input data to perform a characterization of the tissue, and estimate a confidence value for the characterization of the tissue.

By acquiring a first recording, and, if the first recording subceeds a confidence value threshold, acquiring a second recording the reliability of the tissue characterization may be improved.

By acquiring a second reading only if needed, the time required to perform the tissue characterization may be decreased. In other words, one modality is used to immediately determine whether a more time consuming, but more accurate analysis is needed.

For instance, if the optical data is acquired in the second measurement, the second recording allows for further examination of for example tissue perfusion or oxygenation, pulsation or tissue type, to improve accuracy of the characterization of the tissue.

Thus, the system allows for fast characterization of tissue using the first sensor measurements. However, the system is not limited to characterization of the tissue based on the first sensor measurement only. Therefore, the first sensor measurement may be complemented by the second sensor measurement as well. The second sensor measurement is only performed when deemed necessary such that a time-consuming analysis is only performed when necessary.

It should be realized that in some applications, sensing of impedance data may be performed first and may allow a fast or efficient characterization of tissue that may be complemented, if necessary, by sensing of optical data. However, in other applications, sensing of optical data may be performed first and may allow a fast or efficient characterization of tissue that may be complemented, if necessary, by sensing of impedance data.

Furthermore, first sensor measurements using the impedance sensing arrangement may acquire a combination of multifrequency bioimpedance recordings with single frequency bioimpedance recordings using a plurality of electrode configurations. Thus, the first sensing arrangement may be used in multiple ways for iteratively acquiring more input data using the first sensing arrangement for enabling accurate characterization of tissue. Then, only when it is determined that tissue characterization may still not be reliably made, further activation of the optical property sensing arrangement may be initiated, and an accurate detection of pulsation and more in-depth examination of tissue characteristics, including clinical information such as perfusion or oxygenation may be provided.

Moreover, the system as described herein advantageously provide a versatile system that may accurately detect and discriminate different tissue types and pathologies by using plurality of electrode configurations and optical configurations.

In addition, the system may have a plurality of electrode configurations and optical configurations, that may be chosen between. This increases the system robustness, and is adaptable to movement of tissue and suboptimal electrode-tissue contact. By analyzing the phase and magnitude of the bioimpedance data electrode locations that may be affected by a tissue movement and/or suboptimal electrode-tissue contact may be identified. This may provide an indication of which optical components should be activated for the optical recording, and thereby further improve accuracy and reliability of the system.

In an example, the system according to the first aspect may be incorporated in a tissue sealing device. Such a device, comprising the system for tissue characterization could advantageously be used during surgery to detect vessels, which are often hidden inside complex bundles comprising various types of other tissues. Thus, tissue characterization may be provided within a tissue sealing device such that separate devices may not be needed for sealing tissue, i.e., preventing flow, such as blood flow, locally through the sealed tissue, and characterization of tissue. Furthermore, the tissue sealing quality may be assessed by obtaining bioimpedance recordings and optical recordings from the tissue under examination. A properly sealed tissue may experience less pulsation, than a non sealed tissue, and may therefore be identified using the system. Furthermore, the sealing of the tissue may change other tissue characteristics such as the resistance or light absorption.

In a further example, the system according to the first aspect may be incorporated into a cuff. The cuff may advantageously be implanted with the body of the subject and arranged around a tissue structure, and thus be used to monitor tissue. For example, for patients having coronary artery disease, CAD, the system may advantageously be used for monitoring the condition of an artery. Thereby, the system may provide the possibility of detecting a potential artery blockage at an early stage, or detecting the existence of CAD, or the location of the stenosis within the artery.

In a further example, the system according to the first aspect may be incorporated into a pair of catheters. The catheters may be configured to extend into tissue or be configured to be arranged on opposite sides of a tissue segment. The bioimpedance sensing arrangement may comprise electrodes arranged in one or both of the catheters for reflective and/or transmissive measurements. The optical property sensing arrangement may comprise optical components in one or both of the catheters for reflective and/or transmissive measurements.

In a further example, the system according to the first aspect may be incorporated into a pill device. Using the proposed approach, multiple recordings at opposite sides of the pill may be acquired to characterize the tissue surrounding the pill.

In a further example, the system according to the first aspect may be incorporated into a multielectrode array that may be used to monitor cell layer integrity.

The system may be configured to be arranged at least partially in the subject. For instance, at least part of the system may be configured to be implanted in the subject, or to be inserted into the subject, or to be swallowed by the subject.

The bioimpedance sensing arrangement may be configured to be arranged in contact with tissue to be characterized. The optical property sensing arrangement may be configured to be arranged in close relation to the tissue to be characterized. Thus, the bioimpedance sensing arrangement and the optical property sensing arrangement may be configured to be arranged in the subject.

However, processing of acquired data may not necessarily be performed in a unit arranged in the subject. Thus, the decision unit and the control unit may be configured to be arranged in the subject, but may alternatively be configured to be arranged externally to the subject and to communicate with the bioimpedance sensing arrangement and the optical property sensing arrangement.

According to an embodiment, the system comprises a bioimpedance sensing arrangement, configured to be arranged within the subject, and configured to sense an impedance of the tissue for forming impedance data, an optical property sensing arrangement, configured to be arranged within the subject, and configured to sense an optical property of the tissue, a decision unit, configured to receive input data, process the input data to perform a characterization of the tissue, and estimate a confidence value for the characterization of the tissue, wherein the input data comprises the impedance data, and a control unit, configured to determine whether the confidence value subceeds a threshold and, based on the determination that the confidence value subceeds the threshold, activate the optical property sensing arrangement for acquiring further information for characterization of the tissue.

After activation of the optical property sensing arrangement an optical recording may be performed.

The input data may thus, after the optical recording, further comprise the optical property data, and the decision unit may further be configured to process the input data comprising the optical property data to perform a characterization of the tissue, and estimate a confidence value for the characterization of the tissue.

By acquiring a bioimpedance reading, and, if the bioimpedance recording subceeds a confidence value threshold, acquiring an optical recording the reliability of the tissue characterization may be improved.

By acquiring an optical reading only if needed, the time required to perform the tissue characterization may be decreased. In other words, one modality is used to immediately determine whether a more time consuming, but more accurate analysis is needed.

The optical recording allows for further examination of tissue perfusion or oxygenation, to better characterize the tissue.

Thus, the system allows for fast characterization of tissue using sensing of impedance data. However, the system is not limited to characterization of the tissue based on impedance data only. Therefore, the sensing of impedance data may be complemented by sensing optical property data as well. The sensing of optical property data is only performed when deemed necessary such that a time-consuming analysis is only performed when necessary.

Furthermore, by combining multifrequency bioimpedance recordings with single frequency bioimpedance recordings using a plurality of electrode configurations, and further activating, only when required, optical property measurements using a plurality of optical component configurations, an accurate detection of pulsation and more in-depth examination of tissue characteristics, including clinical information such as perfusion or oxygenation may be provided.

According to an embodiment, the bioimpedance sensing arrangement comprises a plurality of electrodes.

The plurality of electrodes of the bioimpedance sensing arrangement may be configured to be arranged within the subject, such that the plurality of electrodes are in contact with the tissue of interest. This implies that the electrodes of the bioimpedance sensing arrangement may be configured to output electrical signals into the tissue for interacting the tissue, and further configured to record a response electrical signal as it interacts with the tissue. For instance, the bioimpedance sensing arrangement may be configured to output a current into tissue and to record a resulting voltage drop as the current interacts with the tissue.

The electrodes of the bioimpedance sensing arrangement may be configured to be arranged in different electrode locations in relation to the tissue.

The different electrode locations may refer to different longitudinal electrode locations. For instance, the tissue to be characterized may have a longitudinal extension. The electrode locations may thus be differently arranged in relation to the longitudinal extension of the tissue.

The plurality of electrodes may be arranged in an array, wherein each of the electrodes arranged in an array corresponds to a different electrode location. Electrodes to be used when performing bioimpedance measurements may be dynamically selected based on the electrodes in the array.

Thanks to the bioimpedance sensing arrangement comprising a plurality of electrodes, the possible number of bioimpedance recording configurations is increased, which may advantageously increase the versatility and reliability of the bioimpedance recordings.

According to an embodiment, the optical property sensing arrangement comprises a plurality of optical components.

The plurality of optical components of the optical property sensing arrangement may be configured to be arranged within the subject, such that the plurality of optical components are configured to receive light from the tissue of interest. the optical property sensing arrangement may be configured to output light into the tissue for interacting with the tissue, and further configured to record a response signal as light interacts with the tissue.

The optical components of the optical property sensing arrangement may be configured to be arranged in different optical component locations in relation to the tissue.

The different optical component locations may refer to different longitudinal optical component locations. For instance, the tissue to be characterized may have a longitudinal extension. The optical component locations may thus be differently arranged in relation to the longitudinal extension of the tissue.

The plurality of optical components may be arranged in an array, wherein each of the optical components arranged in an array corresponds to a different optical component location. Optical components to be used when performing optical sensing measurements may be dynamically selected based on the optical components in the array.

Thanks to the optical property sensing arrangement comprising a plurality of optical components, the possible number of optical recording configurations is increased, which may advantageously increase the versatility and reliability of the optical recordings.

It is a realization that the plurality of electrodes and the plurality of optical components may be arranged in the same array, and that an electrode location may correspond to an optical component location.

According to an embodiment, the optical property sensing arrangement comprises a plurality of optical sensors.

Each optical component of the optical property sensing arrangement may comprise a light source being configured to send light into tissue. Each optical component may comprise a light sensor being configured to receive and detect light from the tissue. Each light sensor may form an optical sensor.

According to an embodiment, the optical property sensing arrangement may further comprise an acoustic sensor. Thus, the optical sensing arrangement may comprise optical components and may further comprise an acoustic sensor. In other words, the optical sensing arrangement may comprise components, which may include optical components and/or the acoustic sensor. It is a realization that each component may comprise at least one of a light source, an optical sensor, or an acoustic sensor. However, as an alternative, each component may comprise at least a light source and at least one of an optical sensor or an acoustic sensor. As a further alternative, each component may comprise a light source, an optical sensor or an acoustic sensor.

Each component of the plurality of components may comprise different combinations of at least one of a light source, an optical sensor and an acoustic sensor. For example a first optical component may comprise a light source and a second component may comprise an optical sensor and an acoustic sensor.

According to an embodiment, a first subset of the plurality of electrodes and a first subset of the plurality of optical components may be configured to be associated with a first tissue segment and wherein different subsets of the plurality of electrodes and subsets of the plurality of optical components may be associated with different tissue segments.

The first subset of the plurality of electrodes and the first subset of the plurality of optical components may be configured to be associated with a first tissue segment. A subset of electrodes may comprise at least two electrodes, or preferably at least four electrodes, of the plurality of electrodes for enabling a bioimpedance measurement to be performed on the tissue segment. A subset of the plurality of optical components may include at least one optical component of the plurality of optical sensors for enabling an optical sensing to be performed on the tissue segment.

Different subsets of the plurality of electrodes and subsets of the plurality of optical components may be associated with different tissue segments.

The plurality of electrodes may comprise one or more pre-defined subsets. However, according to an alternative embodiment, the electrodes forming a subset of electrodes may be dynamically selected among the plurality of electrodes.

Similarly, the plurality of optical sensors may comprise one or more pre-defined subsets. However, according to an alternative embodiment, the optical components forming a subset of optical components may be dynamically selected among the plurality of optical components.

The plurality of electrodes and the plurality of optical components may be arranged in a first array. The first array may be configured to be arranged in relation to one side of the tissue segments such that the electrodes arranged in the first array may be brought into contact with the tissue and the optical property sensing arrangement may be arranged for sending light into the tissue and receiving light from the tissue. This implies that the bioimpedance sensing arrangement and the optical property sensing arrangement may be configured for performing reflective measurements.

The plurality of electrodes and the plurality of optical components may alternatively be arranged in a first array and a second array. The first array and the second array may be configured to be arranged opposed each other facing tissue in opposite directions. In other words, the first and the second array may be arranged such that the tissue of interest may be arranged between the first array and the second array, wherein the first array and the second array faces the tissue. Thus, the electrodes arranged in each array may be brought into contact with the tissue. The optical property sensing arrangement may be configured to provide sending of light into the tissue from one side and detecting light from the tissue at another side. However, the optical property sensing arrangement may also or alternatively be configured to provide sending of light into the tissue and detecting of light from the tissue on the same side.

The first subset of the plurality of electrodes associated with the first tissue segment may include electrodes in both the first and the second array. The first subset of the plurality of optical components associated with the first tissue segment may include optical components in both the first and the second array.

Thanks to the electrodes of the bioimpedance sensing arrangement being arranged in two arrays it is possible to perform both transmissive and reflective bioimpedance recordings. Thanks to the optical components of the optical property sensing arrangement being arranged in two arrays it is possible to perform both transmissive and reflective optical recordings.

According to an embodiment, the bioimpedance sensing arrangement may be configured to acquire a single-frequency time-domain recording, wherein the impedance data may comprise information acquired by the single-frequency time-domain recording, and wherein the decision unit may be configured to process the impedance data for determining presence of a variation of the impedance due to pulsation.

The single-frequency time-domain recording may be acquired for a specific amount of time, for recording how the response signal of the bioimpedance measurement varies over time when interacting with the tissue.

The single-frequency time-domain recording may be performed using at least one of a reflective configuration or a transmissive configuration of the bioimpedance sensing arrangement.

The impedance data may be processed using for example a Fast Fourier Transform (FFT). The FFT may reveal if a peak at low frequencies (around 1 Hz) exists in the spectrum. The low-frequency peak may indicate pulsation, and thus the existence of a vessel in the tissue under examination.

Moreover, the low-frequency peak may indicate the existence of an abnormally increased blood flow, which is typical of cancerous tissue such as tumors.

Thanks to the single-frequency time-domain recording changes in the tissue over time may be determined. Such changes may for example be pulsation due to blood flow.

According to an embodiment, the bioimpedance sensing arrangement may be configured to sense an impedance of the tissue for at least a first frequency and a second frequency.

The first frequency may be different from the second frequency.

For example, the first frequency may be in a first range for distinguishing fatty tissue from muscle tissue based on bioimpedance magnitude, and a second frequency may be in a second range for distinguishing fatty tissue from muscle tissue based on bioimpedance phase.

Preferably, the first frequency may be in a first range of 1 kHz -100 kHz and the second frequency may be in a second range of 100 kHz -10 MHz. Even more preferably, the first frequency may be in a first range of 1 kHz -10 kHz and the second frequency may be in a second range of 300 kHz -1 MHz.

This may be referred to herein as a multi-frequency recording or multi-frequency scan, as more than one frequency is used. The multi-frequency recording may comprise at least two different frequencies, however it is a realization that the more frequencies used, the more accurate the characterization may be. This is due to different types of tissues having different dependency of a response signal to a frequency of the bioimpedance measurement.

Thus, according to an embodiment, the bioimpedance sensing arrangement may be configured to sense an impedance of the tissue using a multi-frequency scan including the first frequency and the second frequency, wherein the bioimpedance sensing arrangement is configured to perform at least one of a reflective measurement or a transmissive measurement at each frequency of the multifrequency scan.

According to an embodiment, the bioimpedance sensing arrangement may be configured to sense the impedance of the tissue in a plurality of iterations, wherein the decision unit is configured to estimate the confidence value after each iteration, and wherein the control unit is configured to, based on the determination that the confidence value subceeds the threshold, first check whether a set maximum of iterations have been performed and, on a condition that a number of performed iterations is lower than the set maximum, activate another iteration.

The bioimpedance sensing arrangement is configured to sense the impedance of the tissue in a plurality of iterations. Each iteration may comprise different electrode configurations, amplitude, frequency step, or frequency range. It is a realization that between each iteration at least one of the listed settings may be changed, however, in an alternative example all of the listed settings are changed.

The maximum number of iterations may be set based on a plurality of factors.

In one example the maximum number of iterations may be set based on the number of different electrode configurations that the system can provide for transmissive and reflective measurements, respectively.

In one example the maximum number of iterations may be set based on the number of different current (or voltage) values that may be used.

In one example the maximum number of iterations may be set based on the number of different frequencies that may be used.

A combination of the above provided examples may be used to set the maximum number of iterations.

The decision unit may be configured to estimate the confidence value after each iteration, and the control unit may be configured to, based on the determination that the confidence value subceeds the threshold, first check whether a set maximum of iterations have been performed. If the number of performed iterations is lower than the set maximum number of iterations, the control unit is further configured to activate another iteration.

According to an embodiment, the control unit may be configured to, in the first sensor measurement, activate the bioimpedance sensing arrangement, and the control unit may further be configured to, on a condition that the set number of iterations of sensing the impedance of the tissue have been performed, activate the optical property sensing arrangement. Thus, if the number of performed iterations has reached the set maximum number of iterations, the control unit is configured to activate the optical property sensing arrangement.

The maximum number of iterations may be utilized to reach the optimal bioimpedance settings.

Thanks to this described iteration process, high reliability and accurate characterization may be achieved, while at the same time keeping the time required to perform the tissue characterization low.

According to an embodiment, the optical property sensing arrangement may be configured to sense an optical property of the tissue for at least a first wavelength and a second wavelength.

The first wavelength may be different from the second wavelength. The first wavelength and the second wavelength may be any (two) wavelengths in the visible spectrum or the near infrared spectrum. Preferably, the first wavelength may be a wavelength corresponding to blue or green light, and the second wavelength may be a wavelength corresponding to red or near infrared light.

For example, the first wavelength may be in a first range of 495-570 nm and the second wavelength may be in a second range of 625-2500 nm. The optical measurement may be at least one of a baseline recording (DC component) or a pulsatile recording (AC component).

Optical properties that may be used to discriminate fatty tissue from vascular tissue may be the AC component of any wavelength from the blue to near infrared spectrum using at least one of a reflective or transmissive configuration, or the DC component in reflective set-up, especially when using wavelengths around a green part of the electro-magnetic spectrum.

The transmissive configuration is sensitive to the tissue width and exerted pressure, whereas a reflective set-up with lower wavelengths (e.g. green light, for which there is low absorption by water) can characterize the tissue to a tissue width of the penetration depth. Both set-ups are capable of returning blood waveform characteristics with a high level of detail.

According to an embodiment, the optical property sensing arrangement may be configured to sense the optical property of the tissue in a plurality of iterations, wherein the decision unit is configured to estimate the confidence value after each iteration, and wherein the control unit is configured to, based on the determination that the confidence value subceeds the threshold, first check whether a set maximum of iterations have been performed and, on a condition that a number of performed iterations is lower than the set maximum, activate another iteration, and, on a condition that the set maximum of iterations have been performed, deactivate the system.

Similarly as for the bioimpedance sensing arrangement, the optical property sensing arrangement is configured to sense the optical property of the tissue in a plurality of iterations. Each iteration may comprise different optical component configurations and wavelengths. It is a realization that between each iteration at least one of the listed settings may be changed, however, in an alternative example all of the listed settings are changed.

The maximum number of iterations may be set based on a plurality of factors. In one example the maximum number of iterations may be set based on the number of different optical component configurations that the system can provide for transmissive and reflective measurements, respectively.

In one example the maximum number of iterations may be set based on the number of different wavelengths that may be used.

A combination of the above provided examples may be used to set the maximum number of iterations.

The decision unit may be configured to estimate the confidence value after each iteration, and the control unit may be configured to, based on the determination that the confidence value subceeds the threshold, first check whether a set maximum of iterations have been performed. If the number of performed iterations is lower than the set maximum number of iterations, the control unit is further configured to activate another iteration. If the number of performed iterations has reached the set maximum number of iterations, the control unit may deactivate the system.

The maximum number of iterations may be utilized to reach the optimal optical settings.

Thanks to this described iteration process, high reliability and accurate characterization may be achieved, while at the same time keeping the time required to perform the tissue characterization low.

According to a second aspect, there is provided a method for controlling a system for characterization of tissue of a subject, the method comprising, initiating a first sensor measurement and activating a first sensing arrangement selected from the bioimpedance sensing arrangement and the optical property sensing arrangement, receiving input data from the first sensing arrangement, processing the input data to perform a characterization of the tissue, estimating a confidence value for the characterization of the tissue, determining whether the confidence value subceeds a threshold, controlling activation of a second sensing arrangement, selected from the bioimpedance sensing arrangement and the optical property sensing arrangement, for acquiring further information for characterization of the tissue based on the determination that the confidence value subceeds the threshold, wherein the second sensing arrangement is non-selected in the first sensor measurement.

This aspect may generally present the same or corresponding advantages as the former aspect. Effects and features of this second are largely analogous to those described above in connection with the first aspect. Embodiments mentioned in relation to the second aspect are largely compatible with the first aspect.

Thanks to the method according to the second aspect, accurate characterization of tissue of a subject is provided in a time-efficient manner.

According to an embodiment, the method comprising, receiving input data, wherein the input data comprises impedance data relating to an impedance of the tissue, processing the input data to perform a characterization of the tissue, estimating a confidence value for the characterization of the tissue, determining whether the confidence value subceeds a threshold, and controlling activation of an optical property sensing arrangement for acquiring further information for characterization of the tissue based on the determination that the confidence value subceeds the threshold.

According to an embodiment, receiving input data may comprise receiving input data comprising impedance data from a single-frequency time-domain recording, and receiving input data comprising impedance data from a multi-frequency measurement for at least a first frequency and a second frequency. The first frequency may be different from the second frequency.

Preferably, the first frequency may be in a first range of 1 kHz -100 kHz and the second frequency may be in a second range of 100 kHz -10 MHz. Even more preferably, the first frequency may be in a first range of 1 kHz -10 kHz and the second frequency may be in a second range of 300 kHz -1 MHz.

According to an embodiment, the impedance data from the multifrequency measurement may comprise a phase value and a magnitude value for each frequency, wherein processing the input data comprises calculating at least one of a phase ratio based on phase values for different frequencies or a magnitude ratio based on the magnitude value of the first frequency and a reference magnitude.

The phase value and the magnitude value may be suitable values that may be extracted from the multifrequency measurement for providing an accurate characterization of tissue.

However, according to an alternative, processing the input data may comprise processing impedance data based on a machine-learned model, wherein the machine-learned model may provide a characterization of tissue based on data received by the machine-learned model. The processing of the input data may comprise extracting features of the impedance data, such as features of the multifrequency measurement, wherein the extracted features are provided to the machine-learned model.

For example, the machine-learned model may provide a classifier algorithm for characterizing the tissue.

According to an embodiment, estimating the confidence value comprises estimating the confidence value based on the calculated at least one of the phase ratio or the magnitude ratio.

This may provide a suitable estimation of the reliability of the characterization of the tissue.

According to an alternative, estimating the confidence value may comprise receiving a confidence value from the machine-learned model. Thus, if a machine-learned model is used for processing the input data, the machine-learned model may also provide an indication of the reliability of the characterization of the tissue output by the machine-learned model.

According to an embodiment, the method further comprises controlling a selection of a subset of electrodes from a plurality of electrodes of the bioimpedance sensing arrangement, wherein the received input data comprises impedance data from the selected subset of electrodes.

This implies that arrangement of the plurality of electrodes in relation to tissue may be facilitated. The plurality of electrodes may not need to be accurately arranged in an exact position. Rather, a subset of electrodes that is arranged in a suitable position for characterization of tissue may be dynamically selected.

According to an embodiment, the method may further comprise, based on the determination that the confidence value subceeds the threshold, first check whether a set maximum of iterations of bioimpedance measurements have been performed and, on a condition that a number of performed iterations is lower than the set maximum, controlling activation of another iteration of bioimpedance measurement, and, on a condition that the set maximum of iterations have been performed, controlling the activation of the optical property sensing arrangement.

The maximum number of iterations may be set based on a plurality of factors.

In one example the maximum number of iterations may be set based on the number of different electrode configurations that the system can provide for transmissive and reflective measurements.

In one example the maximum number of iterations may be set based on the number of different current (or voltage) values that can be injected to the tissue.

In one example the maximum number of iterations may be set based on the number of different frequencies that can be injected to the tissue.

A combination of the above provided examples may be used to set the maximum number of iterations.

According to an embodiment, the method further comprises controlling a selection of a subset of optical components from a plurality of optical components of the optical property sensing arrangement.

According to an embodiment, controlling activation of the optical property sensing arrangement further comprises controlling activation of the subset of optical components.

According to an embodiment, the method further comprises receiving input data from at least a first wavelength and a second wavelength.

Different tissues may comprise different optical properties, and thus by using a first wavelength and a second wavelength, wherein the first wavelength is different from the second wavelength, different tissue types may be discriminated from each other.

The first wavelength and the second wavelength may be any (two) wavelengths in the visible spectrum or the near infrared spectrum. Preferably, the first wavelength may be a wavelength corresponding to blue or green light, and the second wavelength may be a wavelength corresponding to red or near infrared light.

For example, the first wavelength may be in a first range of 495-570 nm and the second wavelength is in a second range of 625-2500 nm.

Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.

Fig. 1 is a schematic view illustrating a system for characterization of tissue of a subject.
Fig. 2a is a schematic view illustrating a detailed view of a bioimpedance sensing arrangement and an optical property sensing arrangement.
Fig. 2b is a schematic view illustrating a detailed view of a system having a transmissive configuration for bioimpedance recordings.
Fig. 2c is a schematic view illustrating a detailed view of a system having a transmissive configuration for optical recordings.
Fig. 2d is a schematic view illustrating a detailed view of a system having a reflective configuration for bioimpedance recordings.
Fig. 2e is a schematic view illustrating a detailed view of the system having a reflective configuration for optical recordings.
Fig. 2f is a schematic view illustrating a detailed view of a system having a transmissive configuration for optical recordings.
Fig. 3 is a schematic view illustrating a system for characterization of tissue of a subject according to an example embodiment.
Fig. 4 is a schematic view illustrating a system for characterization of tissue of a subject according to an example embodiment.
Fig. 5 is a schematic view illustrating a system for characterization of tissue of a subject according to an example embodiment.
Fig. 6 is a schematic view illustrating a system for characterization of tissue of a subject according to an example embodiment.
Fig. 7 is a schematic illustration of a method for characterization of tissue of a subject.
Fig. 8 is a schematic flowchart providing details of use of a system such as the system illustrated in Fig. 1 or Fig. 3-6.

Detailed description

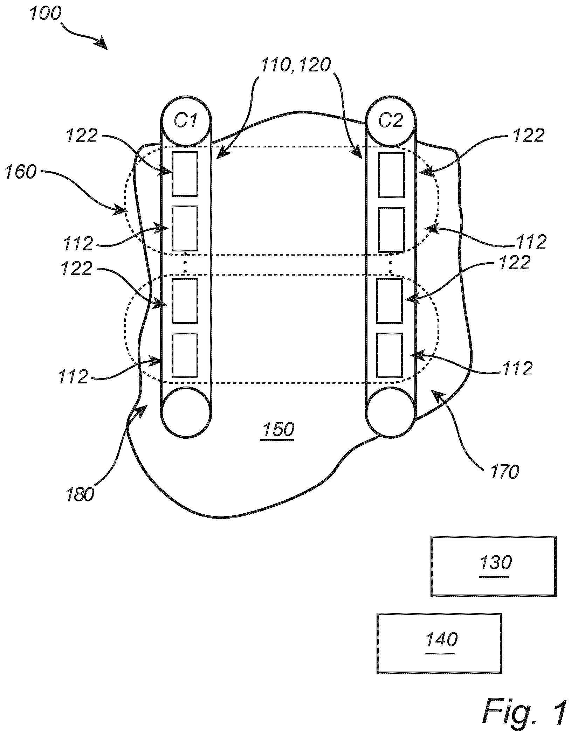

Referring now to Fig. 1, a system 100 for characterization of tissue 150 of a subject will be described.

As illustrated in Fig. 1, the system 100 comprises a bioimpedance sensing arrangement 110, an optical property sensing arrangement 120, a decision unit 130, and a control unit 140.

The bioimpedance sensing arrangement 110 may comprise a plurality of electrodes 112. The electrodes 112 of the plurality of electrodes are preferably arranged in a first array and a second array.

The optical property sensing arrangement 120 may comprise a plurality of optical components 122. The optical components 122 of the plurality of optical components are preferably arranged in a first array and a second array.

In Fig. 1, the bioimpedance sensing arrangement is illustrated having a plurality of electrodes 112, in this case four electrodes 112. The optical property sensing arrangement is illustrated having a plurality of optical components 122, in this case four optical components 122.

While an even number of electrodes 112 are illustrated, it is a realization that an uneven number of electrodes may be used, and that while an even number of optical components 122 are illustrated, it is a realization that an uneven number of optical components may be used. Furthermore, the bioimpedance sensing arrangement and the optical property sensing arrangement does not need to comprise the same number of electrodes or optical components, respectively.

The decision unit 130 may be connected to the bioimpedance sensing arrangement 110 and/or the optical property sensing arrangement 120. The connection may be a wired connection or a wireless connection. The decision unit 130 may for instance be configured to receive input data, wherein the input data may comprise at least one of bioimpedance data from the bioimpedance sensing arrangement 110 or optical data from the optical property sensing arrangement 120.

The decision unit 130 may further be configured to process the input data to perform a characterization of the tissue 150 and to estimate a confidence value for the characterization of the tissue 150.

The control unit 140 may be connected to the bioimpedance sensing arrangement 110 and/or the optical property sensing arrangement 120. The connection may be a wired connection or a wireless connection.

The control unit 140 may further be connected to the decision unit 130. The connection may be a wired connection or a wireless connection.

It should be realized that, while illustrated as separate physical units, the decision unit 130 and the control unit 140 may be implemented in the same physical unit. In other words, a single physical unit may act as both decision unit 130 and a control unit 140.

The control unit 140 may further be configured to determine whether the confidence value subceeds a threshold and, based on the determination that the confidence value subceeds the threshold, activate the optical property sensing arrangement.

The decision unit 130 and the control unit 140 may for instance be implemented in a general-purpose processing unit, such as a central processing unit (CPU), which may execute instructions of one or more computer programs in order to implement functionality of the decision unit 130 and the control unit 140, respectively. However, the decision unit 130 and the control unit 140 may alternatively be implemented as firmware arranged e.g., in an embedded system, or as a specifically designed processing unit, such as an Application-Specific Integrated Circuit (ASIC) or a Field-Programmable Gate Array (FPGA).

It should be further realized that the decision unit 130 and the control unit 140 may be implemented in separate processing units or in a common processing unit.

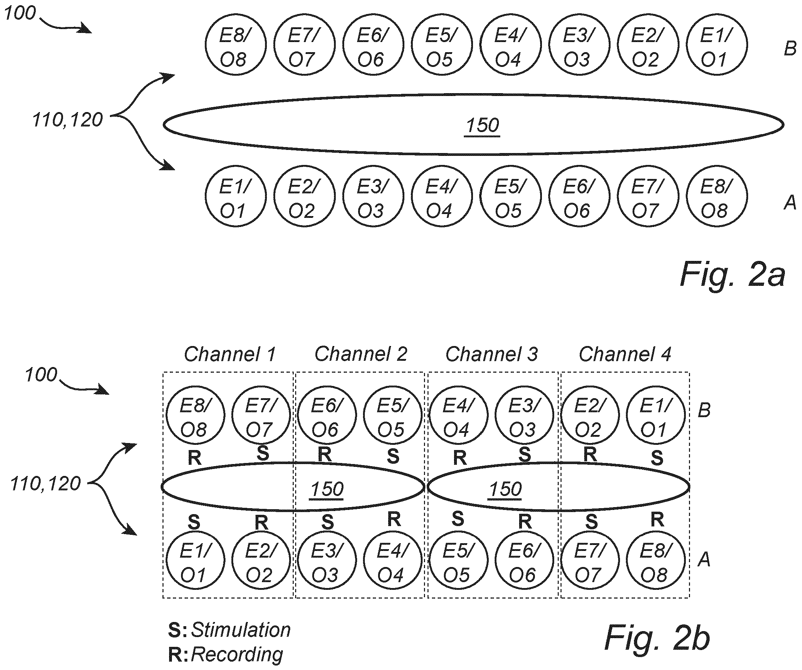

Referring now to Fig. 2a, a bioimpedance sensing arrangement 110 having a plurality of electrodes 112 and an optical property sensing arrangement 120 having a plurality of optical components 122 will be described. Fig. 2b-d will further exemplify different electrode configuration and optical components configurations. It is a realization that these provided illustrated examples are not exhaustive, and only serves the purpose of facilitating understanding of the different possible configurations.

In relation to the following figures (Fig. 2a-2d) the optical property sensing arrangement will be described as comprising optical components comprising a light source and an optical sensor. It should be realized that the provided examples may be applicable for components comprising a light source and an acoustic sensor, for detecting soundwaves induced by emitted light leading to thermal expansion and tissue oscillations for a photo-acoustic measurement, in the same exemplifying configurations.

In Fig. 2a, the plurality of electrodes 112 of the bioimpedance sensing arrangement 110 are illustrated in two arrays. In the illustrated case eight electrodes are arranged in a first array, A, and eight electrodes are arranged in a second array, B. The first array A and the second B are arranged on opposite sides of a tissue segment 150.

Each electrode in the first array, A, is denoted E1, E2, ..., En. Each of the electrodes in the second array, B, is denoted E1, E2, ..., En.

The optical property sensing arrangement 120 may comprise optical components including a light source being configured to send light into tissue and a light sensor being configured to receive and detect light from the tissue. Each light sensor may form an optical sensor 122.

The optical components of the optical property sensing arrangement 120 are illustrated in two arrays. In the illustrated case four light sources are arranged in the first array, A, and four optical sensors are arranged in the second array, B. However, in the illustrated case, the optical property sensing arrangement 120 also comprises light sources arranged in the second array, B, and optical sensors 122 arranged in the first array, A,

Each optical component in the first array, A, is denoted 01, 02, ..., On. Each optical component in the second array, B, is denoted 01, 02, ..., On.

In the illustrated example, electrodes E1-E2 and the light source and optical sensor 01-02 are arranged in the same locations, such that measurements made with electrodes E1-E2 and optical sensor 01-02 relate to the same tissue or tissue segment.

Fig. 2b is a schematic view illustrating a detailed view of a system 100 having a transmissive configuration for bioimpedance recordings.

The system 100 is illustrated having a plurality of electrodes 112 of the bioimpedance sensing arrangement 110 and a plurality of optical sensors 122 of the optical property sensing arrangement 120. The electrodes 112 and the optical sensors 122 are illustrated in a first array A and a second array B. The first array A and the second array B are arranged on opposite sides of a tissue segment 150 to be characterized such that the tissue segment 150 is provided between the first array A and the second array B.

In the transmissive configuration of bioimpedance recordings, a current signal may be provided to travel through the tissue and a voltage drop may be recorded. The voltage drop recorded corresponds to bioimpedance of the tissue that the current travels through.

In the illustrated example of Fig. 2b, four channels are defined, wherein each channel relates to one transmissive bioimpedance recording.

Turning to channel 1, for a transmissive measurement, current flows from electrode E1 of array A to electrode E7 of array B, and a voltage drop is recorded between electrode E2 of array A and electrode E8 of array B.

Turning to channel 2, for a transmissive measurement, current flows from electrode E3 of array A to electrode E5 of array B, and a voltage drop is recorded between electrode E4 of array A and electrode E6 of array B.

Turning to channel 3, for a transmissive measurement, current flows from electrode E5 of array A to electrode E3 of array B, and a voltage drop is recorded between electrode E6 of array A and electrode E4 of array B.

Turning to channel 4, for a transmissive measurement, current flows from electrode E7 of array A to electrode E1 of array B, and a voltage drop is recorded between electrode E8 of array A and electrode E2 of array B.

In other words, four channels result in four recordings. Each channel corresponds to a tissue segment, and thus, each recording is a recording of a tissue segment.

All electrodes may be configured to be both stimulating and recording. In other words, each of the electrodes of the plurality of electrodes 112 can be chosen to act as stimulating electrodes or as recording electrodes. For example, current may flow from electrode E2 of array A to electrode E8 of array B, and a voltage drop may be recorded between electrode E1 of array A and electrode E7 of array B.

While the above is described in relation to bioimpedance recordings, it is a realization that a similar measurement may be performed for optical recordings. In the transmissive configuration, the light travels through the tissue and an intensity drop of the light may be recorded. The intensity drop recorded corresponds to absorbance of the tissue the light travels through.

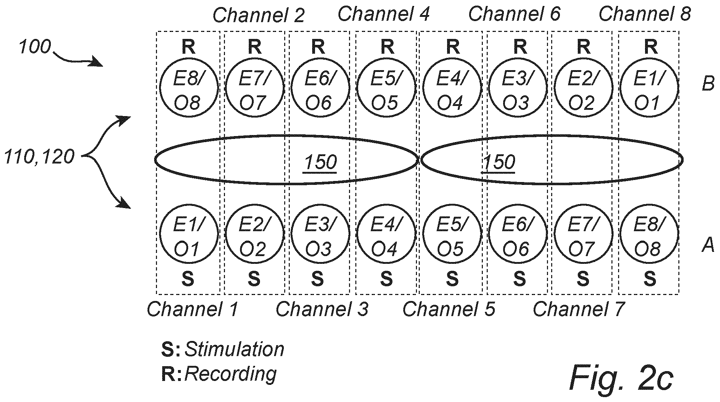

Fig. 2c is a schematic view illustrating a detailed view of a system 100 having a transmissive configuration for optical recordings.

The system 100 is illustrated having a plurality of electrodes 112 of the bioimpedance sensing arrangement 110 and a plurality of optical components 122 of the optical property sensing arrangement 120. The electrodes 112 and the optical components 122 are illustrated in a first array A and a second array B. The first array A and the second array B are arranged on opposite sides of a tissue segment 150 to be characterized such that the tissue segment 150 is provided between the first array A and the second array B.

In the transmissive configuration of bioimpedance recordings, a current signal may be provided to travel through the tissue and a voltage drop may be recorded. The voltage drop recorded corresponds to bioimpedance of the tissue that the current travels through.

In the illustrated example of Fig. 2c, the optical components 122 of the optical property sensing arrangement 120 is arranged such that eight optical channels are defined in corresponding positions to the channels for the bioimpedance recordings such that the eight optical channels may acquire optical recordings for the same tissue segments.

In the transmissive configuration the light propagates through the tissue and a drop in intensity for the light is recorded. The intensity drop recorded corresponds to an optical property of the tissue the light travels through.

The intensity drop may be recorded by using a light sensor, or by using an acoustic sensor configured to detect soundwaves caused by the tissue absorbing light corresponding to the intensity drop.

In particular, two types of recordings may be relevant, a static/slow varying (DC) baseline recording and a pulsatile (AC) recording.

The DC component represents the baseline level of light absorption by the tissue. The DC component is relatively constant over time and represents the overall absorption due to non-pulsatile components like skin, bone, and non-pulsatile blood volume and may be useful for understanding the average light absorption and can provide information about the general composition and thickness of the tissue.

The AC component may represent the pulsatile changes in light absorption due to the cardiac cycle. The AC component varies with each heartbeat, reflecting the changes in blood volume in the microvascular bed of the tissue, and may be used for measuring dynamic physiological parameters like heart rate and blood oxygen saturation.

Turning to channel 1, for a transmissive measurement, light propagates from light source O1 of array A to optical sensor O8 of array B, and the DC component and AC component is recorded representing optical properties between the light source O1 of array A and the optical sensor O8 of array B.

Turning to channel 2, for a transmissive measurement, light propagates from light source O2 of array A to optical sensor O7 of array B, and the DC component and AC component is recorded representing optical properties between the light source O2 of array A and the optical sensor O7 of array B.

Turning to channel 3, for a transmissive measurement, light propagates from light source O3 of array A to optical sensor O6 of array B, and the DC component and AC component is recorded representing optical properties between the light source O3 of array A and the optical sensor O6 of array B.

Tuning to channel 4, for a transmissive measurement, light propagates from light source O4 of array A to optical sensor O5 of array B, and the DC component and AC component is recorded representing optical properties between the light source O4 of array A and the optical sensor O5 of array B.

Turning to channel 5, for a transmissive measurement, light propagates from light source O5 of array A to optical sensor O4 of array B, and the DC component and AC component is recorded representing optical properties between the light source O5 of array A and the optical sensor O4 of array B.

Turning to channel 6, for a transmissive measurement, light propagates from light source O6 of array A to optical sensor O3 of array B, and the DC component and AC component is recorded representing optical properties between the light source O6 of array A and the optical sensor O3 of array B.

Turning to channel 7, for a transmissive measurement, light propagates from light source O7 of array A to optical sensor O2 of array B, and the DC component and AC component is recorded representing optical properties between the light source O7 of array A and optical sensor O2 of array B.

Turning to channel 8, for a transmissive measurement, light propagates from light source O8 of array A to optical sensor O1 of array B, and the DC component and AC component is recorded representing optical properties between the light source O8 of array A and the optical sensor O1 of array B.

Each channel corresponds to a tissue segment, and thus, each DC recording is a recording of a tissue segment and each AC recording is a recording of a tissue segment.

Comparing Fig. 2b and Fig.2c it is a realization that channel 1 in Fig. 2b corresponds to the same tissue segment as channel 1 and channel 2 of Fig. 2c. Further, channel 2 in Fig. 2b corresponds to the same tissue segment as channel 3 and channel 4 of Fig. 2c. Further, channel 3 in Fig. 2b corresponds to the same tissue segment as channel 5 and channel 6 of Fig. 2c. Lastly, channel 4 in Fig. 2b corresponds to the same tissue segment as channel 7 and channel 8 of Fig. 2c.

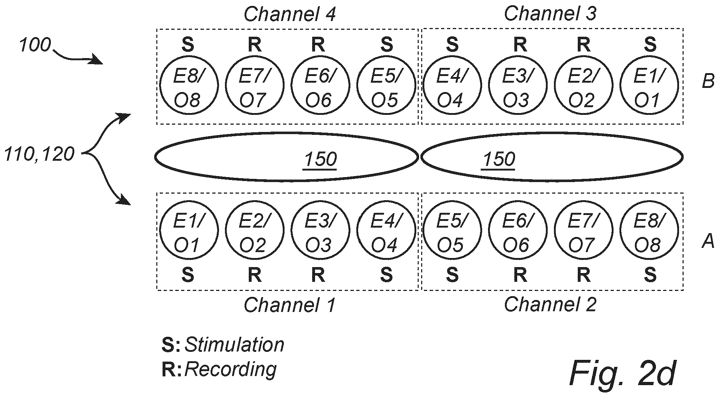

Fig. 2d: Reflective bioimpedance configuration

Fig. 2d is a schematic view illustrating a detailed view of a system 100 having a reflective configuration.

The system 100 is illustrated having a plurality of electrodes 112 of the bioimpedance sensing arrangement 110 and a plurality of optical sensors 122 of the optical property sensing arrangement 120. The electrodes 112 and the optical sensors 122 are illustrated in a first array A and a second array B. The first array A and the second array B are arranged on opposite sides of a tissue segment 150 to be characterized such that the tissue segment 150 is provided between the first array A and the second array B.

In the reflective configuration of bioimpedance recordings, a current signal may be provided to travel along a surface of the tissue and a voltage drop is recorded. The voltage drop recorded corresponds to bioimpedance of the tissue that the current travels through.

In the illustrated example of Fig. 2d, four channels are defined, wherein each channel relates to one reflective bioimpedance recording.

Turning to channel 1, for a reflective measurement, current flows from electrode E1 of array A to electrode E4 of array A, and a voltage drop is recorded between electrode E2 of array A and electrode E3 of array A.

Turning to channel 2, for a reflective measurement, current flows from electrode E5 of array A to electrode E8 of array A, and a voltage drop is recorded between electrode E6 of array A and electrode E7 of array A.

Turning to channel 3, for a reflective measurement, current flows from electrode E4 of array B to electrode E1 of array B, and a voltage drop is recorded between electrode E3 of array B and electrode E2 of array B.

Turning to channel 4, for a reflective measurement, current flows from electrode E8 of array B to electrode E5 of array B, and a voltage drop is recorded between electrode E7 of array B and electrode E6 of array B.

In other words, four channels result in four recordings. Each channel corresponds to a tissue segment, and thus, each recording is a recording of a tissue segment.

As previously described, all electrodes are configured to be both stimulating and recording. In other words, each of the electrodes of the plurality of electrodes 112 can be chosen to act as stimulating electrodes or as recording electrodes. For example, current may flow from electrode E2 of array A to electrode E5 of array A, and a voltage drop may be recorded between electrode E3 of array A and electrode E4 of array A.

While the above is described in relation to bioimpedance recordings, it is a realization that a similar measurement may be performed for optical recordings. In the reflective configuration, the light travels into the tissue and is reflected by diffuse reflection. The intensity of diffusely reflected light corresponds to reflection of the tissue that the light travels through.

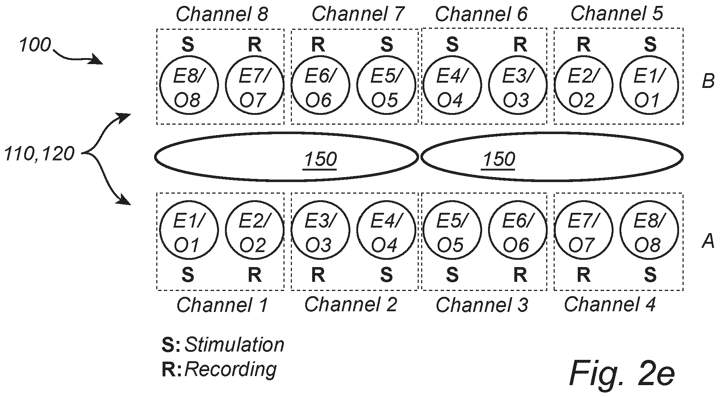

In the illustrated example of Fig. 2e, the components 122 of the optical property sensing arrangement 120 is arranged such that eight optical channels are defined in corresponding positions to the channels for the bioimpedance recordings such that the eight optical channels may acquire optical recordings for the same tissue segments. The components 122 of the optical property sensing arrangement 120 are illustrated in a first array A and a second array B. The first array A and the second array B are arranged on opposite sides of a tissue segment 150 to be characterized such that the tissue segment 150 is provided between the first array A and the second array B.

In the reflective configuration the light is mainly reflected close to the surface of the tissue and a DC component and AC component of the light signal is recorded.

Turning to channel 1, for a reflective measurement, light propagates from lights source O1 of array A to optical sensor O2 of array A, and the DC component and AC component is recorded representing optical properties of tissue at a location of the light source O1 of array A and the optical sensor O2 of array A.

Turning to channel 2, for a reflective measurement, light propagates from lights source O3 of array A to optical sensor O4 of array A, and the DC component and AC component is recorded representing optical properties of tissue at a location of the light source O3 of array A and the optical sensor O4 of array A.

Turning to channel 3, for a reflective measurement, light propagates from light source O5 of array A to optical sensor O6 of array A, and the DC component and AC component is recorded representing optical properties of tissue at a location of the light source O5 of array A and the optical sensor O6 of array A.

Turning to channel 4, for a reflective measurement, light propagates from light source O7 of array A to optical sensor O8 of array A, and the DC component and AC component is recorded representing optical properties of tissue at a location of the light source O7 of array A and the optical sensor O8 of array A.

Turning to channel 5, for a reflective measurement, light propagates from light source O1 of array B to optical sensor O2 of array B, and the DC component and AC component is recorded representing optical properties of tissue at a location of the light source O1 of array B and optical sensor O2 of array B.

Turning to channel 6, for a reflective measurement, light propagates from light source O3 of array B to optical sensor O4 of array B, and the DC component and AC component is recorded representing optical properties of tissue at a location of the light source O3 of array B and optical sensor O4 of array B.

Turning to channel 7, for a reflective measurement, light propagates from light source O5 of array B to optical sensor O6 of array B, and the DC component and AC component is recorded representing optical properties of tissue at a location of the light source O5 of array B and optical sensor O6 of array B.

Turning to channel 8, for a reflective measurement, light propagates from light source O7 of array B to optical sensor O8 of array B, and the DC component and AC component is recorded representing optical properties of tissue at a location of the light source O7 of array B and optical sensor O8 of array B.

Each channel corresponds to a tissue segment, and thus, each AC recording is a recording of a tissue segment and each DC recording is a recording of a tissue segment.

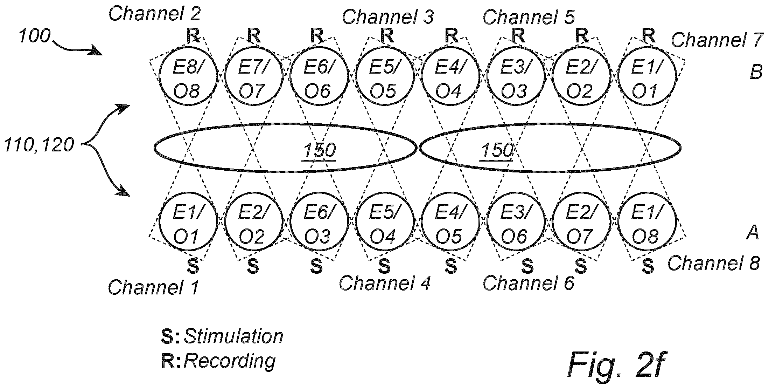

Fig. 2f is a schematic view illustrating a detailed view of a system 100 having a transmissive configuration for optical recordings. The components 122 of the optical property sensing arrangement 120 are illustrated in a first array A and a second array B. The first array A and the second array B are arranged on opposite sides of a tissue segment 150 to be characterized such that the tissue segment 150 is provided between the first array A and the second array B.

Turning to channel 1, for a transmissive measurement, light propagates from lights source O1 of array A to optical sensor O7 of array B, and the DC component and AC component is recorded representing optical properties of tissue at a location of the light source O1 of array A and the optical sensor O7 of array B.

Turning to channel 2, for a transmissive measurement, light propagates from lights source O2 of array A to optical sensor O8 of array B, and the DC component and AC component is recorded representing optical properties of tissue at a location of the light source O2 of array A and the optical sensor O8 of array B.

Turning to channel 3, for a transmissive measurement, light propagates from lights source O3 of array A to optical sensor O5 of array B, and the DC component and AC component is recorded representing optical properties of tissue at a location of the light source O3 of array A and the optical sensor O5 of array B.

Turning to channel 4, for a transmissive measurement, light propagates from lights source O4 of array A to optical sensor O6 of array B, and the DC component and AC component is recorded representing optical properties of tissue at a location of the light source O4 of array A and the optical sensor O6 of array B.

Turning to channel 5, for a transmissive measurement, light propagates from lights source O5 of array A to optical sensor O3 of array B, and the DC component and AC component is recorded representing optical properties of tissue at a location of the light source O5 of array A and the optical sensor O3 of array B.

Turning to channel 6, for a transmissive measurement, light propagates from lights source O6 of array A to optical sensor O4 of array B, and the DC component and AC component is recorded representing optical properties of tissue at a location of the light source O6 of array A and the optical sensor O4 of array B.

Turning to channel 7, for a transmissive measurement, light propagates from lights source O7 of array A to optical sensor O1 of array B, and the DC component and AC component is recorded representing optical properties of tissue at a location of the light source O7 of array A and the optical sensor O1 of array B.

Turning to channel 8, for a transmissive measurement, light propagates from lights source O8 of array A to optical sensor O2 of array B, and the DC component and AC component is recorded representing optical properties of tissue at a location of the light source O8 of array A and the optical sensor O2 of array A.

It is a realization that the above provided configuration, as illustrated in Fig. 2 f, is an example, and other configurations are possible. For example, light may propagate from lights source O1 of array A to optical sensor O6 of array B, and the DC component and AC component is recorded representing optical properties of tissue at a location of the light source O1 of array A and the optical sensor O6 of array B. As a further example, light may propagate from lights source O3 of array B to optical sensor O7 of array A, and the DC component and AC component is recorded representing optical properties of tissue at a location of the light source O3 of array B and the optical sensor O7 of array A.

It is a realization that a electrode configuration or a optical component configuration must not activate and/or utilize all electrodes or optical components.

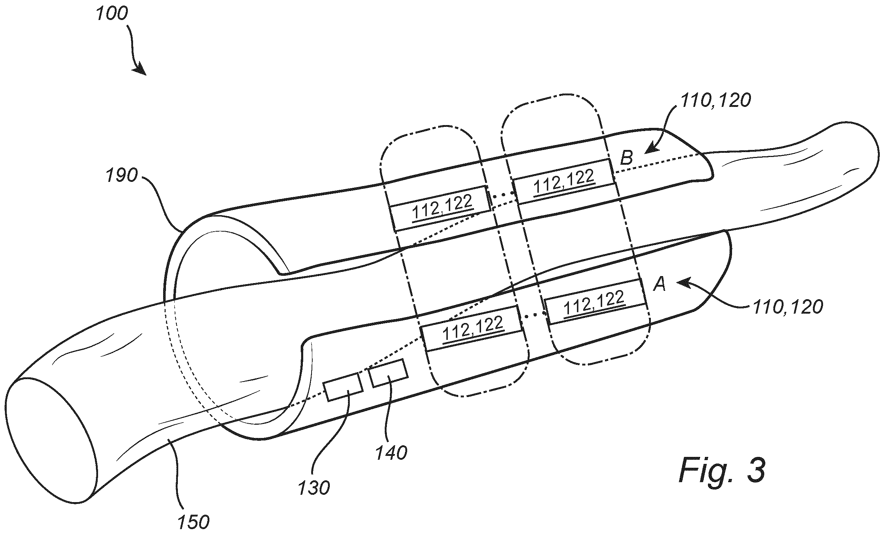

Referring now to Fig. 3, a system 100 for characterization of tissue of a subject according to an example embodiment will be described.

As illustrated in Fig. 3, the system 100 comprises a bioimpedance sensing arrangement 110, an optical property sensing arrangement 120, a decision unit 130, and a control unit 140.

The system illustrated in Fig. 3 is arranged on a cuff 190 and configured to be implanted in the subject, such that the cuff 190 at least partly surrounds an artery or a tissue segment.

The optical property sensing arrangement comprises a plurality of optical components 122, arranged in a first array A and a second array B. The bioimpedance sensing arrangement comprises a plurality of electrodes 112 arranged in a first array A and a second array B.

In the illustrated example, the decision unit 130 and the control unit 140 are arranged on the cuff 190.

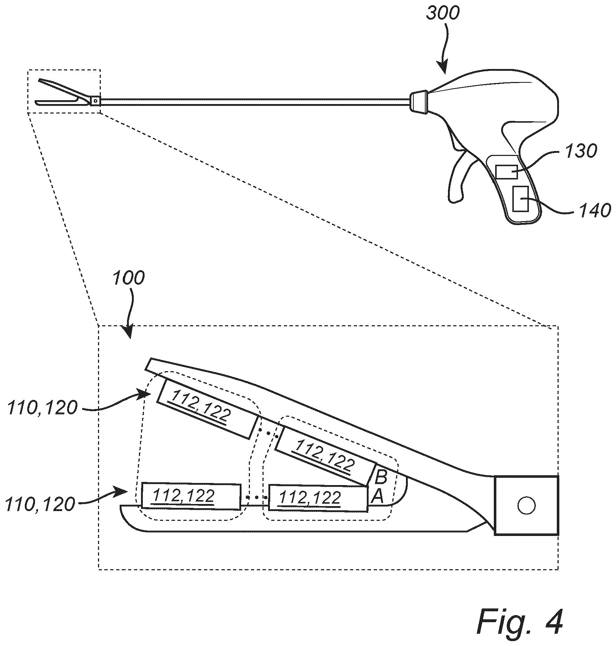

Referring now to Fig. 4, a system 100 for characterization of tissue of a subject according to an example embodiment will be described.

As illustrated in Fig. 4, the system 100 comprises a bioimpedance sensing arrangement 110, an optical property sensing arrangement 120, a decision unit 130, and a control unit 140.

The system 100 is illustrated as part of a tissue sealing device 300. The bioimpedance sensing arrangement 110 comprises a plurality of electrodes 112 and the optical property sensing arrangement 120 comprises a plurality of optical components 122, and are illustrated embedded into the two jaws A,B of a surgical tissue sealing device 300 to acquire bioimpedance recordings and optical recordings during sealing of the tissue. The surgical tissue sealing device 300 may be used for clamping a blood vessel between the jaws A, B in order to prevent blood flow through the blood vessel.

The bioimpedance sensing arrangement 110 and the optical property sensing arrangement 120 may be used for acquiring recordings for ascertaining that the blood vessel is properly sealed, i.e., that no blood flow occurs.

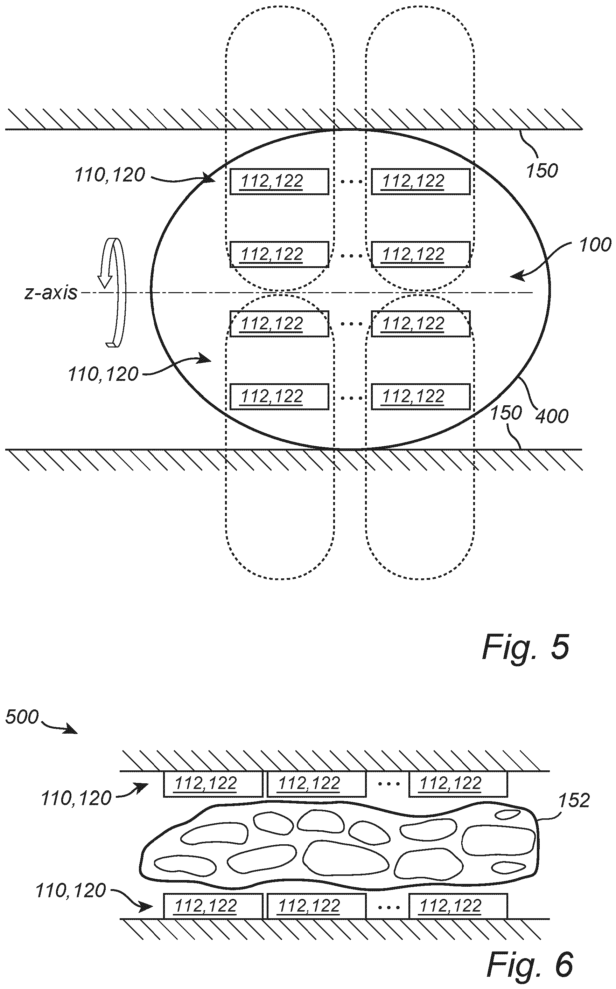

As illustrated in Fig. 5, the system 100 comprises a bioimpedance sensing arrangement 110 and an optical property sensing arrangement 120.

The system may further comprise a decision unit 130, and a control unit 140, not illustrated in fig. 5.

The system 100 is illustrated as part of a pill device 400. The pill device 400 may be configured to arranged within a hollow or open system in the human body, for example within arteries and intestines.

The pill device 400 may have the shape of an ellipsoid, for example a sphere, or as illustrated in Fig.5, a spheroid. The pill device 400 comprising the system 100 may be configured to be able to rotate around an axis Z, thereby characterizing the tissue 150 surrounding the pill device.

In the illustrated example, the pill device 400 comprises the bioimpedance sensing arrangement 110 and the optical property sensing arrangement 120 arranged in four arrays. The bioimpedance sensing arrangement 110 comprises a plurality of electrodes 112 and the optical property sensing arrangement 120 comprises a plurality of optical components 122.

A first and a second array are arranged in a first half of the pill device, and a third and fourth array are arranged in a second half of the pill device. This ensures that characterization may be performed on tissue on two opposite sides of the pill device. However, the bioimpedance sensing arrangement 110 and the optical property sensing arrangement 120 may be arranged in a first array and a second array in the pill device, or in a single array, still having the possibility to perform characterization of the tissue 150 surrounding the pill device thanks to the rotation of the pill device.

According to an alternative, the bioimpedance sensing arrangement 110 and the optical property sensing arrangement 120 may be arranged along an outer surface of the pill device, such that the plurality of electrodes and plurality of optical components at least partly encircle the outer surface of the pill. In this alternative, the tissue surrounding the pill device may be characterized by selectively activating different electrode configurations and optical component configurations, thereby providing the possibility that characterization may be performed on tissue 150 surrounding the pill device in all directions.

The bioimpedance sensing arrangement 110 and the optical property sensing arrangement 120 in the pill device 400 may be configured to use a reflective configuration for characterizing tissue.

Referring now to Fig. 6 the system 100 is shown incorporated into a multielectrode array 500 that may be used to monitor cell layer integrity. As illustrated in Fig. 6, the system 100 comprises a bioimpedance sensing arrangement 110 and an optical property sensing arrangement 120. The bioimpedance sensing arrangement 110 comprises a plurality of electrodes 112 and the optical property sensing arrangement 120 comprises a plurality of optical components 122.

The multielectrode array is shown comprising both the bioimpedance sensing arrangement 110, and the optical property sensing arrangement 120, and may be positioned in close contact with a cell layer 152, as illustrated, and may be used for acquiring recordings for ascertaining the integrity of the cell layer.

In the illustrated example, a first and a second multielectrode array comprising both electrodes of the bioimpedance sensing arrangement 110, and optical components (sensors and light sources) of the optical property sensing arrangement 120, may be arranged opposite each other such that the cell layer 152 may be located between the first and second multielectrode array 500, to provide the possibility of acquiring both transmissive and reflective recordings.

The system may further comprise a decision unit 130, and a control unit 140, not illustrated in Fig. 6.

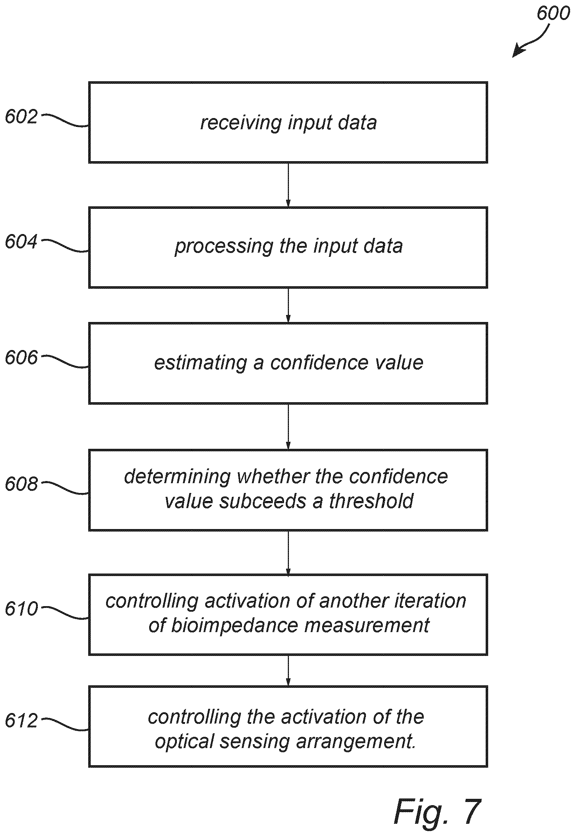

Referring now to Fig. 7, a schematical illustration of a method 600 for characterization of tissue of a subject is shown.

As indicated by block 602, the method 600 comprises receiving input data, wherein the input data comprises impedance data relating to an impedance of the tissue, and, as indicated by block 604, the method 600 further comprises processing the input data to perform a characterization of the tissue. Receiving input data may comprise receiving input data comprising impedance data from a single-frequency time-domain recording, and receiving input data comprising impedance data from a multi-frequency measurement for at least a first frequency and a second frequency.

The processing the input data may comprise calculating at least one of a phase ratio based on phase values for different frequencies or a magnitude ratio based on the magnitude value of the first frequency and a reference magnitude.

As indicated by block 606, the method 600 further comprises estimating a confidence value for the characterization of the tissue, and, as indicated by block 608, determining whether the confidence value subceeds a threshold.

Thereafter, the method may further comprise, based on the determination that the confidence value subceeds the threshold, first check whether a set maximum of iterations of bioimpedance measurements have been performed and, on a condition that a number of performed iterations is lower than the set maximum, controlling activation of another iteration of bioimpedance measurement 610, and, on a condition that the set maximum of iterations have been performed, controlling the activation of the optical property sensing arrangement 612 for acquiring further information for characterization of the tissue based on the determination that the confidence value subceeds the threshold.

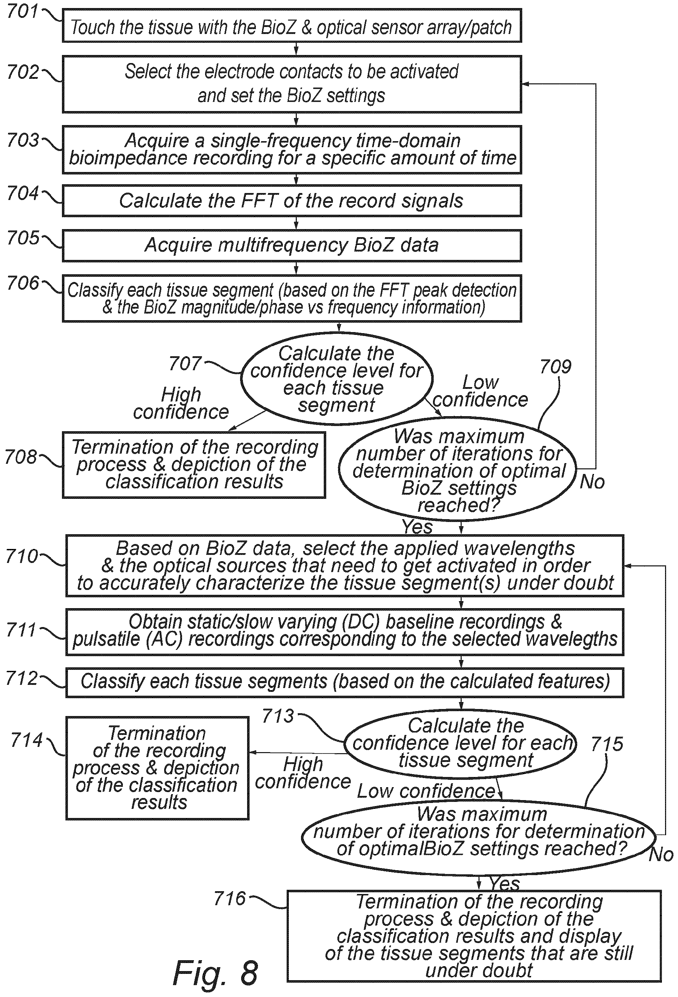

Referring now to Fig. 8, a flowchart is provided, detailing an example use of a system such as the system 100 illustrated in Fig. 1 or Fig. 3-6.

In this provided example of use, the bioimpedance sensing arrangement is selected as the first sensing arrangement selected, and accordingly, the optical property sensing arrangement is selected as the second sensing arrangement. It should be realized that, in other embodiments, the optical property sensing arrangement may be selected as the first sensing arrangement and the bioimpedance sensing arrangement may be selected as the second sensing arrangement. However, for brevity, description is provided below only for the case of the bioimpedance sensing arrangement being selected as the first sensing arrangement.

As indicated by block 701, the bioimpedance sensing arrangement and optical property sensing arrangement may be brought into contact with a tissue to be examined. As disclosed in relation to Fig. 1 and Fig. 3-6 this may be done in a multitude of ways, for example by implantation using a cuff, by swallowing a pill-like device, by inserting catheters, etc. It is a realization that while the way the bioimpedance sensing arrangement and optical property sensing arrangement may be brought into contact with the tissue to be examined may differ depending on the location of the tissue of interest, the electrodes of the bioimpedance sensing arrangement should be at least substantially in direct contact with the tissue and the optical sensors of the optical property sensing arrangement should be configured to receive light from the tissue.

Next, as indicated by block 702, a subset of electrodes from the plurality of electrodes of the bioimpedance sensing arrangement may be selected. The subset may comprise all electrodes of the plurality of electrodes, or at least two electrodes to allow for a first bioimpedance measurement.

The settings for a bioimpedance measurement are chosen. These settings may include the amplitude of the applied current or voltage, the stimulation frequency range, stimulation frequency step, a time to perform the recording, and the stimulation frequency for the single frequency time domain recording.

A single frequency time domain bioimpedance recording is acquired during the chosen time period, as indicated by block 703.

The data retrieved from the single frequency time domain bioimpedance recording is received by the decision unit, and the decision unit processes the data by performing a Fast Fourier Transformation, indicated by block 704. The Fast Fourier Transform (FFT) of the recorded signal is calculated to explore whether a peak at low frequencies (around 1 Hz) exists in the spectrum. Such a peak could reveal pulsation, and thus may reveal the existence of a vessel in the tissue under examination.

As indicated by block 705, a multifrequency bioimpedance data recording is performed, and bioimpedance data is acquired.

The data retrieved from the multifrequency bioimpedance recording is received by the decision unit, and the decision unit processes the data.

Next, each tissue segment is classified, i.e., characterized, by the decision unit extracting appropriate features from the recorded bioimpedance data, based on the FFT peak detection of the single frequency time domain recording, and the bioimpedance magnitude and phase and frequency of the multifrequency bioimpedance recording, as indicated by block 706.

The multifrequency recording corresponds to a recording using at least two different frequencies.

The decision unit may further be configured to calculate a phase ratio. The phase ratio may for example be used to discriminate fatty tissue from vascular tissue.

The phase ratio may for example be defined as the ratio between the mean phase values from frequency recordings from 300kHz up to 1 MHz and the mean phase values from frequency recordings from 2kHz up to 10kHz.

$$Phase\ ratio\ = \frac{Mean\{phase\ values\ from\ 300kHz\ up\ to\ 1MHz\}}{Mean\{phase\ values\ from\ 2kHz\ up\ to\ 10kHz\}}$$

The decision unit may further be configured to calculate a magnitude ratio. The magnitude ratio may for example be used to discriminate fatty tissue from vascular tissue.

The magnitude ratio may for example be defined as the ratio between the magnitude at 2kHz and a reference magnitude, wherein the reference magnitude may be set to a lowest expected magnitude value. For example, the lowest expected magnitude value may be 50 Ohms.

$$Magnitude\ ratio\ = \frac{Magnitude\ at\ 2kHz}{Reference\ magnitude}$$

As indicated by block 707, the decision unit estimates the confidence value of each of the tissue segments, and the control unit determines whether the confidence value subceeds a set threshold.

The decision unit may be configured to estimate the confidence value based on a calculated at least one of the phase ratio or the magnitude ratio. For example, if at least one of the phase ratio and the magnitude ratio is lower than expected pre-defined values, the confidence value may be considered to be low. If the phase ratio and the magnitude ratio is higher than the specific pre-defined values, the confidence value may be considered to be high.

Moreover, the confidence value may be calculated based on the characterization of tissue. For example, if the tissue is characterized as fatty tissue, the confidence value may be calculated as the mean of the ratio between the magnitude ratio and a reference magnitude ratio, and the ratio between the phase ratio and a reference phase ratio.

The reference magnitude ratio may be set to the maximum expected magnitude ratio. The reference phase ratio may be set to the maximum expected phase ratio.

$$Confidence\ value\ = mean\left\{\frac{Magnitude\ ratio}{Reference\ magnitude\ ratio}, \frac{Phase\ ratio}{Reference\ phase\ ratio}\right\}$$

If the tissue is characterized as vascular tissue, the confidence value may be calculated as the mean of the ratio between a reference magnitude and the magnitude ratio, and the ratio between a reference phase ratio and the phase ratio.

The reference magnitude ratio may be set to the minimum expected magnitude ratio. The reference phase ratio may be set to the minimum expected phase ratio.

$$Confidence\ value = mean\left\{\frac{Reference\ magnitude\ ratio}{Magnitude\ ratio}, \frac{Reference\ phase\ ratio}{Phase\ ratio}\right\}$$

In the case wherein the confidence value exceeds the set threshold the recording process is terminated. In other words, the classification is determined to have a high level of reliability, and no additional recordings are needed, indicated by block 708.

In the case wherein the confidence value subceeds the set threshold, a check is made whether a set maximum number of iterations of bioimpedance measurements have been performed, indicated by block 709.

In the case where the set maximum number of iterations of bioimpedance measurements have not yet been reached, blocks 702 through 707 is repeated. In other words, new settings for the amplitude of the stimulation current/voltage, the stimulation frequency range/step, a time to perform the recording, and the stimulation frequency for the single frequency time domain recording are chosen, as well as a new subset of electrodes. Not all settings must be changed at once. For example, it may be determined that it is sufficient to only change the amplitude and nothing else.

In the case where the set maximum number of iterations of bioimpedance measurements have been reached, the control unit will activate the optical property sensing arrangement, indicated by block 710. This may include selecting a subset of optical sensors from the plurality of optical sensors of the optical property sensing arrangement. The subset may comprise all the optical sensors of the plurality of optical sensors, or at least two optical sensors to allow for an optical measurement.

The settings for an optical measurement are chosen. These settings may include the wavelengths to be used in optical measurement.

As indicated by block 711, a static/slow varying baseline recording, also referred to as a DC recording, and a pulsatile recording, also referred to as an AC recording is obtained for each of the wavelengths.

Following this, the tissue segments are characterized again, this time based on the optical recordings, indicated by block 712, and the confidence value for each tissue segment is estimated by the decision unit, indicated by block 713, and the control unit determines whether the confidence value subceeds a set threshold.

In the case wherein the confidence value exceeds the set threshold the recording process is terminated. In other words, the classification is determined to have a high level of reliability, and no additional recordings are needed, indicated by block 714.

In the case wherein the confidence value subceeds the set threshold it is checked whether a set maximum number of iterations of optical measurements have been performed, indicated by block 715.

In the case where the set maximum number of iterations of optical measurements have not yet been reached, blocks 710 through 713 is repeated. In other words, new wavelengths are chosen, and the recordings are repeated.

In the case where the set maximum number of iterations of optical measurements have been reached, the recordings are terminated, indicated by block 716. A characterization of tissue may be provided with an indication that the characterization of tissue may not be fully reliable. Alternatively, an indication that the tissue could not be characterized may be output.

In the above the inventive concept has mainly been described with reference to a limited number of examples. However, as is readily appreciated by a person skilled in the art, other examples than the ones disclosed above are equally possible within the scope of the inventive concept, as defined by the appended claims.

**Claims**

1. A system (100) for characterization of tissue of a subject, the system comprising:

   a bioimpedance sensing arrangement (110), configured to be arranged within the subject, and configured to sense an impedance of the tissue for forming impedance data;
   an optical property sensing arrangement (120), configured to be arranged within the subject, and configured to sense an optical property of the tissue for forming optical property data;
   a control unit (140), configured to initially initiate a first sensor measurement, wherein the control unit is configured to, in the first sensor measurement, activate a first sensing arrangement selected from the bioimpedance sensing arrangement and the optical property sensing arrangement; and

a decision unit (130), configured to receive input data from the first sensing arrangement, process the input data to perform a characterization of the tissue, and estimate a confidence value for the characterization of the tissue; wherein the control unit (140), is further configured to determine whether the confidence value subceeds a threshold and, based on the determination that the confidence value subceeds the threshold, initiate a second sensor measurement, wherein the control unit is configured to, in the second sensor measurement, activate a second sensing arrangement selected from the bioimpedance sensing arrangement and the optical property sensing arrangement for acquiring further information for characterization of the tissue, wherein the second sensing arrangement is non-selected in the first sensor measurement.

2. The system according to claim 1, wherein the bioimpedance sensing arrangement (110) comprises a plurality of electrodes (112), and wherein the optical property sensing arrangement (120) comprises a plurality of components (122).

3. The system according to claim 2, wherein each of the plurality of components of the optical property sensing arrangement (120) comprises at least one of a light source, an optical sensor, or an acoustic sensor.

4. The system according to claim 2 and 3, wherein a first subset of the plurality of electrodes (112) and a first subset (160) of the plurality of optical components (122) are configured to be associated with a first tissue segment and wherein different subsets of the plurality of electrodes and subsets of the plurality of optical sensors are associated with different tissue segments, and

wherein the plurality of electrodes and the plurality of optical components are arranged in a first array (170) and a second array (180) and wherein the first array and the second array are configured to be arranged opposed each other facing tissue in opposite directions and wherein the first subset of the plurality of electrodes associated with the first tissue segment includes electrodes in both the first and the second array and the first subset of the plurality of optical components associated with the first tissue segment includes optical components in both the first and the second array.

5. The system according to any one of the preceding claims, wherein the bioimpedance sensing arrangement is configured to acquire a single-frequency time-domain recording, wherein the impedance data comprises information acquired by the single-frequency time-domain recording, and wherein the decision unit is configured to process the impedance data for determining presence of a variation of the impedance due to pulsation.

6. The system according to any one of the preceding claims, wherein the bioimpedance sensing arrangement is configured to sense an impedance of the tissue for at least a first frequency and a second frequency, wherein the first frequency is different from the second frequency.

7. The system according to claim 6, wherein the bioimpedance sensing arrangement is configured to sense an impedance of the tissue using a multi-frequency scan including the first frequency and the second frequency, wherein the bioimpedance sensing arrangement is configured to perform at least one of a reflective measurement or a transmissive measurement at each frequency of the multifrequency scan.

8. A system according to claim 7, wherein the bioimpedance sensing arrangement is configured to sense the impedance of the tissue in a plurality of iterations, wherein the decision unit is configured to estimate the confidence value after each iteration, and wherein the control unit is configured to, based on the determination that the confidence value subceeds the threshold, first check whether a set maximum of iterations have been performed and, on a condition that a number of performed iterations is lower than the set maximum, activate another iteration.

9. A method for controlling a system for characterization of tissue of a subject, the method comprising:

initiating a first sensor measurement and activating a first sensing arrangement selected from the bioimpedance sensing arrangement and the optical property sensing arrangement;
receiving input data from the first sensing arrangement;
processing the input data to perform a characterization of the tissue;
estimating a confidence value for the characterization of the tissue;
determining whether the confidence value subceeds a threshold;
controlling activation of a second sensing arrangement, selected from the bioimpedance sensing arrangement and the optical property sensing arrangement, for acquiring further information for characterization of the tissue based on the determination that the confidence value subceeds the threshold, wherein the second sensing arrangement is non-selected in the first sensor measurement.

10. The method according to claim 9, wherein receiving input data from the first sensing arrangement comprises

receiving input data from the bioimpedance sensing arrangement comprising impedance data from a single-frequency time-domain recording; and
receiving input data from the bioimpedance sensing arrangement comprising impedance data from a multi-frequency measurement for at least a first frequency and a second frequency, wherein the first frequency is different from the second frequency.

11. The method according to claim 10, wherein the impedance data from the multifrequency measurement comprises a phase value and a magnitude value for each frequency, wherein processing the input data comprises calculating at least one of a phase ratio based on phase values for different frequencies or a magnitude ratio based on the magnitude value of the first frequency and a reference magnitude.

12. The method according to claim 11, wherein estimating the confidence value comprises estimating the confidence value based on the calculated at least one of the phase ratio or the magnitude ratio.

13. The method according to claim 9, further comprising controlling a selection of a subset of electrodes from a plurality of electrodes of the bioimpedance sensing arrangement, wherein the received input data comprises impedance data from the selected subset of electrodes.

14. The method according to claim 9, further comprising, based on the determination that the confidence value subceeds the threshold, first check whether a set maximum of iterations of bioimpedance measurements have been performed and, on a condition that a number of performed iterations is lower than the set maximum, controlling activation of another iteration of bioimpedance measurement, and, on a condition that the set maximum of iterations have been performed, controlling the activation of the optical property sensing arrangement.

15. The method according to any one of claim 9-14, further comprising controlling a selection of a subset of optical components from a plurality of optical sensors of the optical property sensing arrangement, and wherein controlling activation of the optical property sensing arrangement further comprises controlling activation of the subset of optical sensors, and wherein the method further comprises receiving input data from at least a first wavelength and a second wavelength.

*100*

*110,120*

C1     C2

*122*

*160*

*122*

*112*

*122*

*112*

*112*

*112*

*150*

*180*

*170*

*130*

*140*

*Fig. 1*

Fig. 2a

Fig. 2b

S: Stimulation
R: Recording

**S:** *Stimulation*
**R:** *Recording*

*Fig. 2c*

Fig. 2d

Fig. 2e

S: Stimulation
R: Recording

EP 4 751 646 A1

Fig. 2f

Fig. 3

Fig. 4

110,120

112,122  · · ·  112,122

112,122  · · ·  112,122

150

100

z-axis

112,122  · · ·  112,122

110,120

112,122  · · ·  112,122

150  400

*Fig. 5*

500

110,120

112,122  112,122  · · ·  112,122

152

110,120

112,122  112,122  · · ·  112,122

*Fig. 6*

*600*

602 — receiving input data

604 — processing the input data

606 — estimating a confidence value

608 — determining whether the confidence value subceeds a threshold

610 — controlling activation of another iteration of bioimpedance measurement

612 — controlling the activation of the optical sensing arrangement.

*Fig. 7*

701 — Touch the tissue with the BioZ & optical sensor array/patch

702 — Select the electrode contacts to be activated and set the BioZ settings

703 — Acquire a single-frequency time-domain bioimpedance recording for a specific amount of time

704 — Calculate the FFT of the record signals

705 — Acquire multifrequency BioZ data

706 — Classify each tissue segment (based on the FFT peak detection & the BioZ magnitude/phase vs frequency information)

707 — Calculate the confidence level for each tissue segment

High confidence

Low confidence — 709

708 — Termination of the recording process & depiction of the classification results

Was maximum number of iterations for determination of optimal BioZ settings reached? — No

Yes

710 — Based on BioZ data, select the applied wavelengths & the optical sources that need to get activated in order to accurately characterize the tissue segment(s) under doubt

711 — Obtain static/slow varying (DC) baseline recordings & pulsatile (AC) recordings corresponding to the selected wavelegths

712 — Classify each tissue segments (based on the calculated features)

713 — Calculate the confidence level for each tissue segment — 715

714 — Termination of the recording process & depiction of the classification results

High confidence

Low confidence

Was maximum number of iterations for determination of optimalBioZ settings reached? — No

Yes

716 — Termination of the recording process & depiction of the classification results and display of the tissue segments that are still under doubt

Fig. 8

Europäisches
Patentamt

European
Patent Office

Office européen
des brevets

# EUROPEAN SEARCH REPORT

**Application Number**

EP 24 21 6698

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/160250 A1 (ANDERSON DAVID A [US] ET AL) 26 May 2022 (2022-05-26) | 1-7,9-15 | INV. A61B5/0537 |
| A | * paragraphs [0046], [0048], [0084] - [0088], [0094]; figures 2,3,7 * | 8 | G16H50/20 A61B5/0538 A61B17/28 |
| A | IT UB20 153 157 A1 (UNIV DEGLI STUDI ROMA LA SAPIENZA) 18 February 2017 (2017-02-18) * pages 9-22; figures 1,2 * | 1-15 | ADD. A61B5/0535 A61B17/29 |
| A | US 2012/296238 A1 (CHERNOV BORIS [RU] ET AL) 22 November 2012 (2012-11-22) * paragraphs [0026] - [0040]; figures 1,2 * | 1-15 | |

**TECHNICAL FIELDS SEARCHED (IPC)**

A61B

The present search report has been drawn up for all claims

| Place of search | Date of completion of the search | Examiner |
|---|---|---|
| The Hague | 11 April 2025 | Mecking, Nikolai |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 21 6698

This annex lists the patent family members relating to the patent documents cited in the above-mentioned European search report.
The members are as contained in the European Patent Office EDP file on
The European Patent Office is in no way liable for these particulars which are merely given for the purpose of information.

11-04-2025

| Patent document cited in search report | | Publication date | Patent family member(s) | | Publication date |
|---|---|---|---|---|---|
| US 2022160250 | A1 | 26-05-2022 | CN | 116471985 A | 21-07-2023 |
| | | | EP | 4247237 A1 | 27-09-2023 |
| | | | US | 2022160250 A1 | 26-05-2022 |
| | | | US | 2025082222 A1 | 13-03-2025 |
| | | | WO | 2022108667 A1 | 27-05-2022 |
| IT UB20153157 | A1 | 18-02-2017 | | | |
| US 2012296238 | A1 | 22-11-2012 | EP | 2709549 A1 | 26-03-2014 |
| | | | US | 2012296238 A1 | 22-11-2012 |
| | | | WO | 2012158777 A1 | 22-11-2012 |

EPO FORM P0459

For more details about this annex : see Official Journal of the European Patent Office, No. 12/82